# EUROPÄISCHE PATENTSCHRIFT

(11) **EP 1 185 499 B1**
(45) Veröffentlichungstag und Bekanntmachung des Hinweises auf die Patenterteilung: **04.02.2004**
(21) Anmeldenummer: 00927211.3
(22) Anmeldetag: 16.05.2000
(51) Int. Cl.: C07C 67/343, C07C 69/734

(54) **VERFAHREN ZUR HERSTELLUNG VON ALKOXYZIMTSÄUREESTER**
METHOD OF PRODUCING ALKOXYCINNAMIC ACID ESTER
PROCEDE DE PRODUCTION D'ESTER D'ACIDE ALCOXYCINNAMIQUE

(30) Priorität: 27.05.1999 DE 19924402; 09.05.2000 DE 10022108
(43) Veröffentlichungstag der Anmeldung: 13.03.2002
(73) Patentinhaber: Symrise GmbH & Co. KG, 37603 Holzminden (DE)
(72) Erfinder: KUHN, Walter, D-37603 Holzminden (DE); MARKS, Werner, D-37647 Brevörde (DE); ZAHLMANN, Klaus, D-37603 Holzminden (DE)
(74) Vertreter: Eisenführ, Speiser & Partner
(86) Internationale Anmeldenummer: PCT/EP2000/004421
(87) Internationale Veröffentlichungsnummer: WO 2000/073255

(56) Entgegenhaltungen:
- US-A- 5 527 947

## Beschreibung

Die Erfindung betrifft die Herstellung von Alkoxyzimtsäureester (Alkoxycinnamaten).

2-Ethylhexyl-4-methoxycinnamat und 3-Methylbutyl-4-methoxycinnamat als auch deren Isomere stellen bekannte und effiziente Lichtschutzmittel für den UV-B-Bereich dar und werden in technischem Umfang hergestellt.

Zur Herstellung dieser Produkte werden Verfahren gesucht, die preiswert sind und mit großen Ausbeuten durchgeführt werden können.

In der US 5 527 947 wird ein Verfahren beschrieben, bei dem C₁-C₄-Alkoxybenzaldehyde, wie Anisaldehyd, und C₁-C₄-Alkylacetate, wie Methylacetat, in einem inerten Kohlenwasserstoff wie Heptan, Toluol oder Petrolether gelöst werden. In Gegenwart einer stark alkalischen Metallbase, wie Natriummethylat, reagieren die Einsatzstoffe zu einem Gemisch der entsprechenden C₁-C₄-Alkyl-C₁-C₄-Alkoxycinnamate, der Alkalimetallsalze der entsprechenden C₁-C₄-Alkoxyzimtsäuren und der C₁-C₄-Alkanole. Nach Ansäuern des Reaktionsgemisches mit einer starken mehrbasigen Säure, wie Schwefelsäure, wird die freigesetzte Essigsäure mit den C₁-C₄-Alkanolen verestert und abdestilliert. Das verbleibende Gemisch aus C₁-C₄-Alkyl-C₁-C₄-Alkoxycinnamaten und C₁-C₄-Alkoxyzimtsäuren wird mit einem C₅-C₁₄-Alkanol in Gegenwart eines Metallsalzes einer starken mehrbasigen Säure wie Schwefelsäure umgesetzt. Nach Umesterung und Veresterung wird das entsprechende C₅-C₁₄-Alkyl-C₁-C₄-alkoxycinnamat, z.B. 2-Ethylhexyl-4-methoxycinnamat mit 83 bis 87 % Ausbeute, erhalten.

Es wurde ein Verfahren zur Herstellung von Zimtsäureestem der Formel 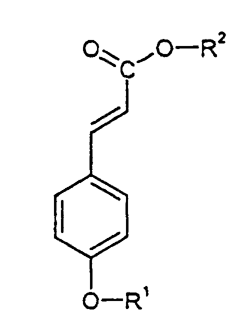 worin
- R¹: für eine Alkylgruppe mit 1 bis 4 Kohlenstoffatomen und
- R²: für 2-Ethylhexyl oder 3-Methylbutyl steht,
gefunden, das dadurch gekennzeichnet ist, dass Essigsäureester der Formel 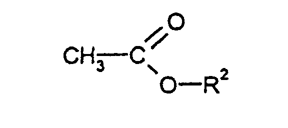 worin R² die oben genannte Bedeutung hat,
mit einem Alkoxybenzaldehyd der Formel 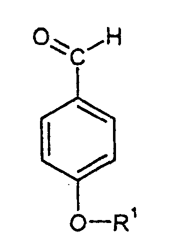 worin R¹ die oben genannte Bedeutung hat,
in Gegenwart eines Alkalialkoholats kondensiert und während der Umsetzung entstehender Alkohol entfernt wird.

Das erfindungsgemäße Verfahren kann durch das folgende Formelschema erläutert werden:

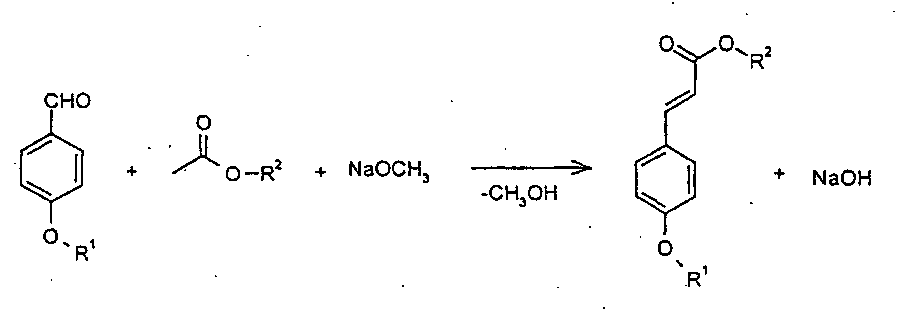

Alkoxybenzaldehyde für das erfindungsgemäße Verfahren können beispielsweise Anisaldehyd, 4-Ethoxybenzaldehyd, 4-Propoxybenzaldehyd, 4-iso-Propoxybenzaldehyd, 4-Butoxybenzaldehyd und 4-iso-Butoxybenzaldehyd sein.

Die Essigsäureester für das erfindungsgemäße Verfahren sind 2-Ethylhexylacetat oder 3-Methylbutylacetat.

Alkalialkoholate für das erfindungsgemäße Verfahren sind bevorzugt die Natrium- und Kaliumalkoholate niederer aliphaltischer Alkohole (C₁ bis etwa C₄) wie Methanol, Ethanol, Propanol, iso-Propanol, Butanol und iso-Butanol. Insbesondere bevorzugt ist Natriummethylat.

Die Essigsäureester können in reiner Form verwendet werden oder im Gemisch mit 2-Ethylhexanol oder 3-Methylbutanol. Um die Wirtschaftlichkeit des Verfahrens zu erhöhen, ist es vorteilhaft, 2-Ethylhexylacetat oder 3-Methylbutylacetat vor bzw. während der Kondensationsreaktion in situ herzustellen und dieses Reaktionsgemisch ohne Reinigung oder Wäschen zur Kondensationsreaktion einzusetzen. Dies kann z.B. geschehen, indem
a) 2-Ethylhexanol oder 3-Methylbutanol mit einem Essigsäure-C₁-C₄-ester wie Methylacetat, Ethylacetat, Propylacetat, Isopropylacetat, Butylacetat oder Isobutylacetat in Gegenwart eines Alkali- und/oder Erdalkalialkoholats zum Einsatzstoff 2-Ethylhexylacetat oder 3-Methylbutylacetat umgeestert wird,
   oder
b) 2-Ethylhexanol oder 3-Methylbutanol mit Essigsäure autokatalytisch oder in Gegenwart katalytischer Mengen einer starken Säure wie Schwefelsäure oder 4-Toluolsulfonsäure verestert und andestilliert werden.

Das unter a) hergestellte Gemisch kann sofort mit einem C₁-C₄-Alkoxybenzaldehyd wie Anisaldehyd zur Kondensationsreaktion gebracht werden.

Das unter b) hergestellte Gemisch wird durch Zugabe von Alkalialkoholat für die Kondensationsreaktion vorbereitet.

Die Zugabe von Alkoxybenzaldehyd geschieht bevorzugt im Temperaturbereich -10 bis 120°C, insbesondere bevorzugt von 10 bis 30°C, innerhalb von 0,5 bis 5 Stunden, insbesondere bevorzugt 1 bis 2 Stunden.

Es wird 0,5 bis 10 Stunden, bevorzugt 1 bis 3 Stunden, nachgerührt, wobei die Reaktionstemperatur auf 50 bis 150°C, bevorzugt 80 bis 110°C erhöht wird. Während des Nachrührens wird ein Vakuum von 800 mbar bis 2 mbar, bevorzugt von 80 mbar bis 40 mbar angelegt.

Der bei dem erfindungsgemäßen Verfahren entstehende Alkohol wird bevorzugt bei der Destillation im Vakuum abgetrennt.

In der Nachrührzeit unter Vakuum wird restlicher Alkoxybenzaldehyd zum 4-Alkoxyzimtsäureester umgesetzt und niedrige homologe 4-Methoxycinnamate zum erfindungsgemäßen Alkoxyzimtsäureester, 2-Ethylhexyl-4-methoxycinnamat oder 3-Methylbutyl-4-methoxycinnamat umgeestert.

Das Reaktionsgemisch wird dann bevorzugt mit einer starken Säure, wie Schwefelsäure, Schwefelsäure/NaHSO₄ oder 4-Toluolsulfonsäure, versetzt. Im Anschluss wird bis 150°C Sumpftemperatur destilliert, wobei die als Nebenprodukt entstandene 4-Methoxyzimtsäure (ca. 20 %) zum erfindungsgemäßen Alkoxyzimtsäureester verestert wird. Gleichzeitig wird entstandene Essigsäure zu 2-Ethylhexylacetat bzw. 3-Methylbutylacetat verestert. Nach Wäsche und Destillation wird 2-Ethylhexyl-4-methoxycinnamat oder 3-Methylbutyl-4-methoxycinnamat mit hoher Ausbeute (90 bis 93 %) und hoher Reinheit (96 bis 98 %) bei einfacher Destillation erhalten.

Die bei der Destillation anfallenden Zwischenfraktionen 2-Ethylhexanol/2-Ethylhexylacetat oder 3-Methylbutanol/3-Methylbutylacetat können bei der nächsten Kondensationsreaktion wieder mitverwendet werden.

Die Vorteile des erfindungsgemäßen Verfahrens sind:
- Einsatz von preiswerten Rohstoffen
- Minimierung der Abfälle und Wiederverwendung von Nebenläufen
- Minimierung der organischen Fracht im Abwasser

Es ist überraschend, dass nach dem erfindungsgemäßen Verfahren Alkoxyzimtsäureester in hohen Ausbeuten und in hoher Reinheit durch einfache Destillation hergestellt werden können.

### Beispiele

### Herstellung von 2-Ethylhexyl-4-methoxycinnamat

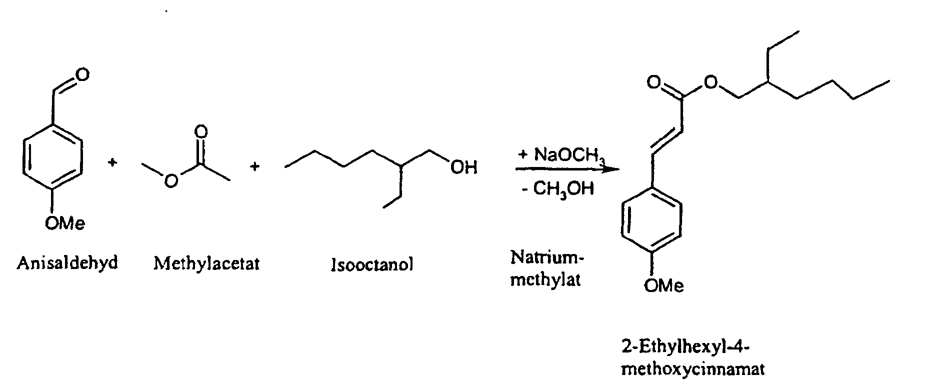

### Beispiel 1: H₂SO₄/NaHSO₄-Variante

In einem 6 l-Doppelmantelgefäß werden
2 106 g 2-Ethylhexanol = 16,20 Mol
1 200 g Methylacetat = 16,20 Mol
187 g Natriummethylat = 3,46 Mol
vorgelegt.

Bei 20 bis 30°C werden in ca. 80 Minuten 440,0 g Anisaldehyd = 3,24 Mol zudosiert. Anschließend wird auf 100°C aufgeheizt, wobei ein Gemisch aus Methanol und Methylacetat abdestilliert. Bei nachlassender Destillation wird bei 60 mbar Vakuum angelegt. Bei 100°C/60 mbar werden in 2 Stunden insgesamt ca. 920 g Leichtsieder abdestilliert. Zur Neutralisation wird bei 100°C in 15 Minuten 262 g 70 %ige Schwefelsäure = 1,87 Mol zudosiert. Danach wird bis zu einer Sumpftemperatur von 145 bis 150°C in 2 Stunden ca. 160 g Wasser ausgekreist. Nach Abkühlen auf <100°C wird mit 700 g Wasser gewaschen. Nach Phasentrennung wird mit 200 g Wasser und 2 g techn. Natronlauge nachgewaschen. Anschließend wird die organische Phase über eine 10 cm Vigreux-Kolonne destilliert.

| Fraktion | Sumpftemperatur | Kopftemperatur/mbar | Gewicht |
|---|---|---|---|
| 1 | 67-200°C | 52-125°C/2,5 | 1910 g (je 40-50 % 2-Ethylhexanol/2-Ethylhexylacetat |
| 2 (HM) | -250°C | -165°C/2,0 | 865 g (97 % 2-Ethylhexyl-4-methoxycinnamat) |

Die Ausbeute beträgt 90 % der Theorie.

### Beispiel 2: 4-Toluolsulfonsäure-Variante

In einem 6 l-Doppelmantelgefäß werden
2 106 g 2-Ethylhexanol = 16,20 Mol
1 200 g Methylacetat = 16,20 Mol
187 g Natriummethylat = 3,46 Mol
vorgelegt.

Bei 20 bis 30°C werden in ca. 80 Minuten 440 g Anisaldehyd = 3,24 Mol zudosiert. Anschließend wird auf 100°C aufgeheizt, wobei ein Gemisch aus Methanol und Methylacetat abdestilliert. Bei nachlassender Destillation wird bis 60 mbar Vakuum angelegt. Bei 100°C/60 mbar werden in 2 Stunden insgesamt ca. 920 g Leichtsieder abdestilliert. Zur Neutralisation wird bei 100°C in 15 Minuten 243 g 70 %ige Schwefelsäure = 1,73 Mol (= äquimolare Menge) zudosiert. Es werden 20 g 4-Toluolsulfonsäure = 3 % bezogen auf Mol Na-Methylat zugegeben.

Weitere Durchführung wie Beispiel 1.

| Fraktion | Sumpftemperatur | Kopftemperatur/mbar | Gewicht |
|---|---|---|---|
| 1 | 64-195°C | 51-111°C/1,5 | 1814 g (je 40 bis 50 % 2-Ethylhexanol/2-Ethylhexylacetat |
| 2 (HM) | -250°C | -165°C/1,0 | 870 g (98 % 2-Ethylhexyl-4-methoxycinnamat) |

Die Ausbeute beträgt 91,5 % der Theorie.

### Beispiel 3: H₂SO₄/NaHSO₄-Variante mit 2-Ethylhexanol/2-Ethylhexylacetat wgw:

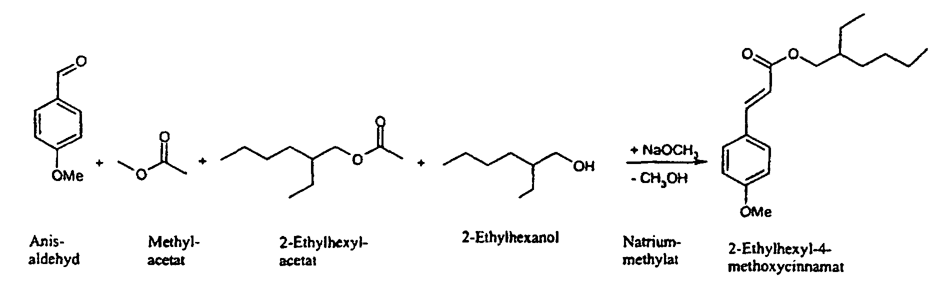

In einem 6 l-Doppelmantelgefäß werden
1685 g 2-Ethylhexanol/2-Ethylhexylacetat wgw.
bestehend aus 675 g = 5,2 Mol 2-Ethylhexanol und = 5,9 Mol 2-Ethylhexylacetat)
480 g 2-Ethylhexanol = 3,7 Mol
720 g Methylacetat = 9,7 Mol
187 g Natriummethylat = 3,46 Mol
vorgelegt.

Bei 20 bis 30°C werden in ca. 80 Minuten 440 g Anisaldehyd = 3,24 Mol zudosiert.

Weitere Durchführung analog Beispiel 1, H₂SO₄-Variante.

| Fraktion | Sumpftemperatur | Kopftemperatur/mbar | Gewicht |
|---|---|---|---|
| 1 | 65-198°C | 48-78°C/1,4-1,7 | 1700 g (38 % 2-Ethylhexanol, 61 % 2-Ethylhexylacetat) |
| 2 (HM) | -265°C. | -170°C/1,6 | 906 g (96,1 % 2-Ethylhexyl-4-methoxycinnamat) |

Die Ausbeute beträgt 92,7 % der Theorie.

## Patentansprüche

1. Verfahren zur Herstellung von Zimtsäureestem der Formel 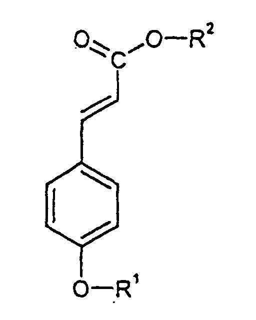 worin
R¹ für eine Alkylgruppe mit 1 bis 4 Kohlenstoffatomen und
R² für 2-Ethylhexyl oder 3-Methylbutyl steht,
**dadurch gekennzeichnet, dass** Essigsäureester der Formel 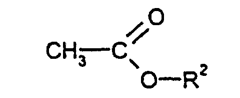 worin R² die oben genannte Bedeutung hat,
mit einem Alkoxybenzaldehyd der Formel 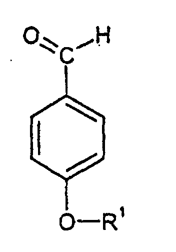 worin R¹ die oben genannte Bedeutung hat,
in Gegenwart eines Alkalialkoholats kondensiert und während der Umsetzung entstehender Alkohol entfernt wird.

2. Verfahren nach Anspruch 1, **dadurch gekennzeichnet, dass** ein Natrium- oder Kaliumalkoholat von Methanol, Ethanol, Propanol iso-Propanol, Butanol oder iso-Butanol eingesetzt wird.

3. Verfahren nach den Ansprüchen 1 und 2, **dadurch gekennzeichnet, dass** als Alkalialkoholat Natriummethanolat eingesetzt wird.

4. Verfahren nach den Ansprüchen 1 bis 3, **dadurch gekennzeichnet, dass** das Molverhältnis der Reaktanten für die Kondensation Essigsäureester : Alkoxybenzaldehyd : Alkalialkoholat 1,5 bis 15 : 1 : 1,0 bis 1,5 beträgt.

5. Verfahren nach den Ansprüchen 1 bis 4, **dadurch gekennzeichnet, dass** das Molverhältnis der Reaktanten für die Kondensation Essigsäureester : Alkoxybenzaldehyd : Alkalialkoholat etwa 5 : 1 : 1,1 beträgt.

6. Verfahren nach den Ansprüchen 1 bis 5, **dadurch gekennzeichnet, dass** die Kondensation im Temperaturbereich von 0 bis 120°C durchgeführt wird.

7. Verfahren nach den Ansprüchen 1 bis 6, **dadurch gekennzeichnet, dass** die Kondensation im Temperaturbereich von 10 bis 30°C durchgeführt wird.

8. Verfahren nach den Ansprüchen 1 bis 7, **dadurch gekennzeichnet, dass** die Kondensation innerhalb von 0,5 bis 10 Stunden durchgeführt wird.

9. Verfahren nach den Ansprüchen 1 bis 8, **dadurch gekennzeichnet, dass** die Kondensation innerhalb von 1 bis 2 Stunden durchgeführt wird.

10. Verfahren nach den Ansprüchen 1 bis 9, **dadurch gekennzeichnet, dass** der entstehende Alkohol während der Kondensation durch ein Vakuum von 800 mbar bis 1 mbar entfernt wird.

11. Verfahren nach den Ansprüchen 1 bis 10, **dadurch gekennzeichnet, dass** der entstehende Alkohol während der Kondensation durch ein Vakuum von 80 mbar bis 40 mbar entfernt wird.

12. Verfahren nach den Ansprüchen 1 bis 11, **dadurch gekennzeichnet, dass** der Essigsäureester in situ hergestellt wird.

13. Verfahren nach den Ansprüchen 1 bis 12, **dadurch gekennzeichnet, dass** der Essigsäureester in situ durch Umsetzung von 2-Ethylhexanol oder 3-Methylbutanol mit Essigsäure-C₁-C₄-ester in Gegenwart eines Alkalialkoholats hergestellt wird

14. Verfahren nach den Ansprüchen 1 bis 12, **dadurch gekennzeichnet, dass** der Essigsäureester in situ durch Umsetzung von 2-Ethylhexanol oder 3-Methylbutano mit Essigsäure autokatalytisch oder in Gegenwart katalytischer Mengen einer starken Säure hergestellt wird.

15. Verfahren nach den Ansprüchen 1 bis 12 und 14, **dadurch gekennzeichnet, dass** als starke Säuren Schwefelsäure/NaHSO₄ oder 4-Toluolsulfonsäure eingesetzt werden.

16. Verfahren nach den Ansprüchen 1 bis 12, **dadurch gekennzeichnet, dass** das Reaktionsprodukt ohne Isolierung mit dem Alkoxybenzaldehyd umgesetzt wird.

17. Verfahren nach den Ansprüchen 1 bis 12, 14 und 15, **dadurch gekennzeichnet, dass** der in situ erzeugte Essigsäureester ohne Isolierung mit dem Alkoholat und dann mit dem Alkoxybenzaldehyd umgesetzt wird.

18. Verfahren nach den Ansprüchen 1 bis 17, **dadurch gekennzeichnet, dass** das Kondensationsprodukt mit einer starken Säure angesäuert wird und vorhandene Alkoxyzimtsäure zum erfindungsgemäßen Alkoxyzimtsäureester verestert wird.

19. Verfahren nach den Ansprüchen 1 bis 18, **dadurch gekennzeichnet, dass** das Verhältnis der Menge der starken Säure zum Alkoholat 0,5 bis 2 : 1 beträgt.

20. Verfahren nach den Ansprüchen 1 bis 19, **dadurch gekennzeichnet, dass** die Zimtsäureester durch Destillation aus dem Reaktionsgemisch isoliert werden.

## Claims

1. Process for the production of cinnamic esters having the formula 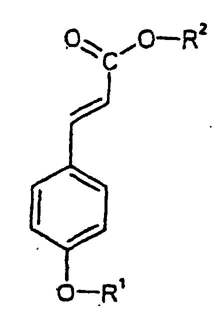 in which
R¹ stands for an alkyl group having 1 to 4 carbon atoms and
R² stands for 2-ethylhexyl or 3-methylbutyl,
**characterised in that** acetic acid ester having the formula 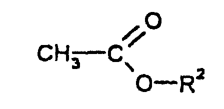 in which R² has the meaning given above,
is condensed with an alkoxybenzaldehyde having the formula 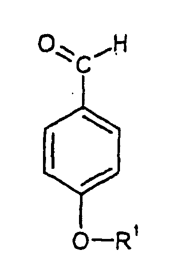 in which R¹ has the meaning give above,
in the presence of an alkali alkoxide and alcohol formed during the reaction is removed.

2. Process according to claim 1, **characterised in that** a sodium or potassium alkoxide of methanol, ethanol, propanol, iso-propanol, butanol or iso-butanol is used.

3. Process according to claims 1 and 2, **characterised in that** sodium methanolate is used as the alkali alkoxide.

4. Process according to claims 1 to 3, **characterised in that** the molar ratio of the reactants for the condensation, acetic acid ester : alkoxybenzaldehyde : alkali alkoxide, is 1.5 to 15 : 1 : 1.0 to 1.5.

5. Process according to claims 1 to 4, **characterised in that** the molar ratio of the reactants for the condensation, acetic acid ester : alkoxybenzaldehyde : alkali alkoxide, is approximately 5 : 1 : 1.1.

6. Process according to claims 1 to 5, **characterised in that** the condensation is performed in the temperature range from 0 to 120°C.

7. Process according to claims 1 to 6, **characterised in that** the condensation is performed in the temperature range from 10 to 30°C.

8. Process according to claims to 1 to 7, **characterised in that** the condensation is performed over a period of 0.5 to 10 hours.

9. Process according to claims to 1 to 8, **characterised in that** the condensation is performed over a period of 1 to 2 hours.

10. Process according to claims 1 to 9, **characterised in that** the alcohol formed during the condensation is removed by a vacuum of 800 mbar to 1 mbar.

11. Process according to claims 1 to 10, **characterised in that** the alcohol formed during the condensation is removed by a vacuum of 80 mbar to 40 mbar.

12. Process according to claims 1 to 11, **characterised in that** the acetic acid ester is prepared in situ.

13. Process according to claims 1 to 12, **characterised in that** the acetic acid ester is prepared in situ by reacting 2-ethylhexanol or 3-methylbutanol with acetic acid C₁-C₄ ester in the presence of an alkali alkoxide.

14. Process according to claims 1 to 12, **characterised in that** the acetic acid ester is prepared in situ by reacting 2-ethylhexanol or 3-methylbutanol with acetic acid autocatalytically or in the presence of catalytic amounts of a strong acid.

15. Process according to claims 1 to 12 and 14, **characterised in that** sulfuric acid/NaHSO₄ or 4-toluenesulfonic acid are used as the strong acids.

16. Process according to claims 1 to 12, **characterised in that** the reaction product is reacted without isolation with the alkoxybenzaldehyde.

17. Process according to claims 1 to 12, 14 and 15, **characterised in that** the acetic acid ester generated in situ is reacted without isolation with the alkoxide and then with the alkoxybenzaldehyde.

18. Process according to claims 1 to 17, **characterised in that** the condensation product is acidified with a strong acid and alkoxycinnamic acid that is present is esterified to give the alkoxycinnamic acid ester according to the invention.

19. Process according to claims 1 to 18, **characterised in that** the ratio of the amount of strong acid to alkoxide is 0.5 to 2 : 1.

20. Process according to claims 1 to 19, **characterised in that** the cinnamic acid esters are isolated from the reaction mixture by distillation.

## Revendications

1. Procédé pour la préparation d'esters d'acide cinnamique de la formule 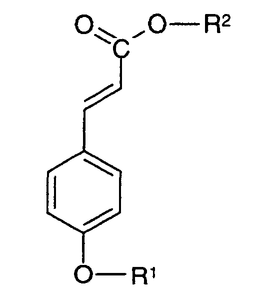 où
R¹ représente un groupe alkyle avec de 1 à 4 atomes de carbone et
R² représente le groupe 2-éthylhexyle ou 3-méthylbutyle,
**caractérisé en ce que** l'on condense un ester d'acide acétique de la formule 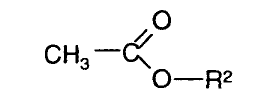 où R² a la signification citée ci-dessus,
avec un alcoxybenzaldéhyde de la formule 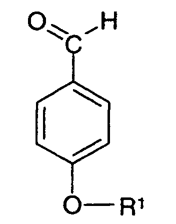 où R¹ a la signification citée ci-dessus,
en présence d'un alcoolate d'alcali et on élimine l'alcool produit pendant la réaction.

2. Procédé selon la revendication 1, **caractérisé en ce que** l'on utilise un alcoolate de sodium ou de potassium du méthanol, de l'éthanol, du propanol, de l'isopropanol, du butanol ou de l'isobutanol.

3. Procédé selon les revendications 1 et 2, **caractérisé en ce que** l'on utilise comme alcoolate d'alcali du méthanolate de sodium.

4. Procédé selon les revendications 1 à 3, **caractérisé en ce que** le rapport molaire des corps réagissants pour la condensation ester d'acide acétique:alcoxybenzaldéhyde:alcoolate d'alcali est de 1,5 à 15:1:1,0 à 1,5.

5. Procédé selon les revendications 1 à 4, **caractérisé en ce que** le rapport molaire des corps réagissants pour la condensation ester d'acide acétique:alcoxybenzaldéhyde:alcoolate d'alcali est d'environ 5:1:1,1.

6. Procédé selon les revendications 1 à 5, **caractérisé en ce que** l'on réalise la condensation dans un domaine de température de 0 à 120°C.

7. Procédé selon les revendications 1 à 6, **caractérisé en ce que** l'on réalise la condensation dans un domaine de température de 10 à 30°C.

8. Procédé selon les revendications 1 à 7, **caractérisé en ce que** l'on réalise la condensation en l'espace de 0,5 à 10 h.

9. Procédé selon les revendications 1 à 8, **caractérisé en ce que** l'on réalise la condensation en l'espace de 1 à 2 h.

10. Procédé selon les revendications 1 à 9, **caractérisé en ce que** l'on élimine l'alcool produit pendant la condensation par un vide de 800 mbar à 1 mbar.

11. Procédé selon les revendications 1 à 10, **caractérisé en ce que** l'on élimine l'alcool produit pendant la condensation par un vide de 80 mbar à 40 mbar.

12. Procédé selon les revendications 1 à 11, **caractérisé en ce que** l'ester d'acide acétique est préparé in situ.

13. Procédé selon les revendications 1 à 12, **caractérisé en ce que** l'ester d'acide acétique est préparé in situ par réaction de 2-éthylhexanol ou de 3-méthylbutanol avec un ester en C₁-C₄ d'acide acétique en présence d'un alcoolate d'alcali.

14. Procédé selon les revendications 1 à 12, **caractérisé en ce que** l'ester d'acide acétique est préparé in situ par réaction de 2-éthylhexanol ou de 3-méthylbutanol avec de l'acide acétique de manière autocatalytique ou en présence de quantités catalytiques d'un acide fort.

15. Procédé selon les revendications 1 à 12 et 14, **caractérisé en ce que** l'on utilise comme acide fort de l'acide sulfurique/NaHSO₄ ou de l'acide 4-toluènesulfonique.

16. Procédé selon les revendications 1 à 12, **caractérisé en ce que** l'on fait réagir le produit de la réaction sans l'isoler avec l'alcoxybenzaldéhyde.

17. Procédé selon les revendications 1 à 12, 14 et 15, **caractérisé en ce que** l'on fait réagir l'ester d'acide acétique produit in situ sans l'isoler avec l'alcoolate et après avec l'alcoxybenzaldéhyde.

18. Procédé selon les revendications 1 à 17, **caractérisé en ce que** le produit de condensation est acidifié avec un acide fort et est estérifié avec l'acide alcoxycinnamique présent pour produire l'ester d'acide alcoxycinnamique selon l'invention.

19. Procédé selon les revendications 1 à 18, **caractérisé en ce que** le rapport de la quantité de l'acide fort à l'alcoolate est de 0,5 à 2:1.

20. Procédé selon les revendications 1 à 19, **caractérisé en ce que** l'ester d'acide cinnamique est isolé du mélange réactionnel par distillation.
